# EUROPEAN PATENT APPLICATION

(11) **EP 2 332 464 A1**
(43) Date of publication of application: **15.06.2011**
(21) Application number: 10015243.8
(22) Date of filing: 02.12.2010
(51) Int. Cl.: A61B 5/117, A61C 5/08, A61C 8/00, A01K 11/00, A61B 19/00, G06K 19/07, G07F 7/00, G06Q 20/00

(54) **Identification device**

(30) Priority: 08.12.2009 TW 09814183
(71) Applicant: Li, Yu-Jung, Sanchong City T'ai pei 241 (TW)
(72) Inventor: Li, Yu-Jung, Sanchong City T'ai pei 241 (TW)
(74) Representative: UEXKÜLL & STOLBERG

(57) **Abstract**

The invention provides an identification device, which comprises a dental implantation carrier and an information storage element therein. The identification device has good privacy and bio-compatibility. The invention also provides a method for identification by using the device.

## Description

### BACKGROUND OF THE INVENTION

### FIELD OF THE INVENTION

The present invention is related to an identification device; particularly to an identification device having good privacy and bio-compatibility, which is located within a body of a subject to be identified.

### DESCRIPTION OF THE RELATED ART

Stray dog is an increasingly serious social issue. In order to prohibit malicious abandonment and to search for missing dogs, the government of Republic of China is the first to legislate in the Animal Protection Act that each dog should be implanted an animal chip. Each the animal chip contains a specific number, which can be read by a reader and input into an administration's database to obtain information of the dog/host.

The conventional method for implanting the animal chip is to subcutaneously inject a chip into an animal's subcutaneous tissues, which is theoretically simple and does not cause much pain so that having good prognosis without significant side effects. In fact, since the chip is located within the subcutaneous tissues, it is inevitable to conduct allergic inflammation or ulcer of skin occurred by bio-compatibility issues and have the risks of being damaged by external force. Thus, the practice of the conventional animal chips is still needed to be improved.

Except for the aforementioned identification of dog/host, identification of human being also has been an important task in human society for a long time. After security incidents or natural disasters, some unidentified bodies are usually found. In the past, the main procedure for identifying those bodies is to inform families having missing members for body inquest. However, it takes too much time and works poorly. Moreover, it is hard to avoid the doubts of inhumanity while considering the sorrow, sadness and uneasy emotion of those families during inquest.

Fingerprint test and dentition test are alternative identification methods. However, the ratio of the population filed in the fingerprint database and the dentition database by the whole population is so low that the fingerprint test and dentition test usually do not work effectively. Moreover, the databases especially lack for child's information so that make the tests fail for searching missing child. Inasmuch as that, some developed countries begin an action to establish a whole population fingerprint database; however, the action is eventually paused because of privacy issues.

In the light of foregoing, identification, no matter for human beings or animals, is no doubt necessary. But how to complete it is a question still needed to be resolved.

### SUMMARY OF THE INVENTION

In view of foregoing, one object of the present invention is to provide an identification device, which is located with a tooth or a bone of the oral cavity of a subject to be identified so that the risk of being damaged and poor prognosis is reduced and bio-compatibility thereof is improved.

Another object of the present invention is to provide an identification device located within a tooth or a bone of the oral cavity of a subject to be identified, that is, the location for said identification device is private so that the privacy of said subject can be protected.

To achieve the above objects, the present invention provides an identification device, comprising a dental implantation carrier; and an information storage element positioned in said implantation carrier.

Preferably, said implantation carrier is a dental crown, a dental post, a dental implant or a bone screw

In one embodiment of the present invention, said identification device comprise a dental crown; and an information storage element positioned by said dental crown.

In one embodiment of the present invention, said identification device comprise a dental post; and an information storage element positioned in said dental post.

In one embodiment of the present invention, said identification device comprise a dental implant; and an information storage element positioned in said dental implant.

In one embodiment of the present invention, said identification device comprise a bone screw; and an information storage element positioned in said bone screw.

Preferably, said information comprises user identification, user personal information or a combination thereof.

Preferably, said information further comprises a remaining sum of electronic money.

Preferably, said information storage element is a chip, a flash memory, a metal-etched code or a combination thereof.

Preferably, said chip is an identification chip; preferably, said chip is a contactless chip, a contact chip or a combination thereof.

Preferably, said information storage element is coated by a metal prosthesis; preferably, said a metal of said metal prosthesis is titanium.

The present invention also provides a method for identification, comprising the following steps: (a) positioning at least one identification device in accordance with claim 1 within an oral cavity of a subject to be identified; and (b) reading an information storage element of said identification device.

Preferably, said identification device is positioned within a tooth or a bone inside said oral cavity.

Preferably, said method further comprising a step of removing said identification device from said oral cavity before said step (b).

Preferably, a type of said reading is contactless-reading or contact-reading.

Preferably, said subject comprises human beings or animals.

To sum up, the present invention is related to an identification device and a method for identification by using the same, wherein a location of said device is at a tooth or a bone of an oral cavity, which is more private than that of conventional devices. Consequently, said identification device is not easy to be known by other people and is not easy to be damaged by external force, and by choosing different types of information storage elements in accordance with the desire of the user, the concern of privacy can be resolved. Furthermore, methods for implanting said device are routine medical operations of dental clinics, which have been already developed. Therefore, the practice of the device of the present invention is easy and almost without any prognosis risks; that is, an acceptance for a subject to implant said device is increased, and the identification issues existed for a long time can be consequently resolved.

### BRIEF DESCRIPTION OF THE DRAWINGS

Figure 1a shows a sectional view of the identification device of example 1 of the present invention.
Figure 1b shows a sectional view of the example of figure 1a after implanting.
Figure 2a shows a sectional view of the identification device of example 2 of the present invention.
Figure 2b shows a sectional view of the example of figure 2a after implanting.
Figure 3a shows a sectional view of the identification device of example 3 of the present invention.
Figure 3b shows a sectional view of the example of figure 3a after implanting.
Figure 4a shows a sectional view of the identification device of example 4 of the present invention.
Figure 4b shows a sectional view of the example of figure 4a after implanting.

### DETAILED DESCRIPTION OF THE PRESENT INVENTION

The present invention is related to an identification device and a method for identification by using the same. Said identification device is designed to equip an information storage element with information into a dental implantation carrier. Said identification device can be located at a location within a tooth or a bone of a subject's oral cavity by using conventional and developed dental operations.

A tooth or a bone (such as an alveolar bone) of a oral cavity is chosen as a location for said device because of its advantages of being highly private, not easy to be damaged by external force and easy to install. Moreover, teeth and bones are relatively bioinert tissues so that are better for implanting chips than subcutaneous tissues, which has high risks of prognosis. In term of privacy, a location of a tooth or a bone in an oral cavity is relatively highly private; therefore, by choosing a proper information storage element, the object of fast identification can be achieved in a balance of privacy. Accordingly, said device is much more suitable than fingerprint test and dentition test for users with privacy concerns.

It is noted that the device of the present invention is coated by a metal prosthesis and/or located inside a dental implantation carrier. In other words, said device does not directly contact with the inner tissues of a user; therefore, it is rare to cause any physiological responses and complies with related medical regulations. Meanwhile, said metal prosthesis is preferably made of titanium with a purity of at least 99%, which therefore has excellent bio-compatibility.

Said information storage element includes, but not limited to: a chip, a flash memory, a metal-etched code or a combination thereof. Said chip is not limited, for instance, a chip traditionally used for subcutaneously injecting to an animal is suitable to be used in the present invention. Besides, said chip can be a contactless chip, a contact chip or a combination thereof in accordance with a user's desire. Said contactless chip is referred to as a chip which can be read without direct contact with a corresponding reader; whereas, said contact chip is referred to as a chip which is read by substantially direct contact with a sensor of a corresponding reader.

Also, a shape of said chip is not limited; preferably, it is chosen in accordance with said implantation carrier's shape, for instance, a short-tube shape. A method for storing information into said chip includes but not limited to a well-known conventional write-in progress for chips and memories (a contact write-in progress) or a RFID system (radio frequency identification, a contactless write-in progress).

Said flash memory is a conventional flash memory without further limitation. Said metal-etched code is obtained by etching a number at a proper area of said information storage element, wherein a length of said number is not limited; preferably, said code has at least seven digits for providing services for more users and having higher security. Each said code is specific for one user and is corresponding by storing the user's information into a database. Accordingly, by entering the code into the database, the information of the user can be obtained. Said metal is not limited; preferably, said metal is titanium with high bio-compatibility. Preferably, said metal-etched code can be used as a back-up choice for identification while said chip or said flash memory is broken or dysfunction.

Said information is user identification, user personal information or a combination thereof and can further comprises a remaining sum of electronic money. Said user identification includes, but not limited to: ID number, name, birthday, etc, which can be used for identifying the user; said personal information includes, but not limited to: blood type, record of dread diseases, willingness of organ donation, emergency contact person, etc; said electronic money includes, but not limited to: EasyCard, iCash, credit card, debit card, etc, for improving life convenience. Taking blood type as an example, based on the conventional process of emergency treatment, when a wounded person, who is in coma or unconscious, is delivered to a hospital and needs blood transfusion, it takes time for blood type examination or accessing information to make sure the blood type thereof. In other word, some precious time for emergency treatment is wasted in this way. On the other hand, if the wounded person has been implanted the identification device of the present invention, medical staffs can read the chip located in the oral cavity of the person at the first time to obtain blood type or record of dread diseases thereof and take the most appropriate treatment immediately. In this way, the efficiency of emergency treatment can be improved effectively.

Said reading is corresponding to the kind of the information storage element used. For instance, if a contactless chip is used, a corresponding reader can be used to read the chip directly from the outside of the user's body. If a contact chip is used, said information storage element is first removed from the device of the present invention and then put into a corresponding reader to read out the stored information. If a metal-etched code is used, said information storage element is first removed from a tooth or bone of the user to identify the code predetermined on said information storage element, and the code is then entered into a database to obtain information.

The following examples are used to further realize the advantages of the present invention but not intended to limit the claim of the present invention. Furthermore, the following drawings are merely used for illustrating the elements of the present invention but not intended to limit the scale of said elements.

### Example 1: Implantation and read of the identification device of the present invention containing a dental crown as an implantation carrier

When a tooth is broken or damaged to a larger extent by reasons such as colliding, it is needed to use a dental crown to substitute the original crown for maintaining the masticatory function of the tooth.

An information storage element used in this example was a flash memory in a short-tube shape. An ID number, name, blood type, emergency contact person and record of dread diseases of the user were stored in said flash memory.

For implanting the device of the present invention: first of all, the original crown of the user was ground to a proper size and used as a support for positioning a dental crown. Then, a mold was sampled on the ground original tooth by known operation, and a dental crown was molded in accordance with said mold.

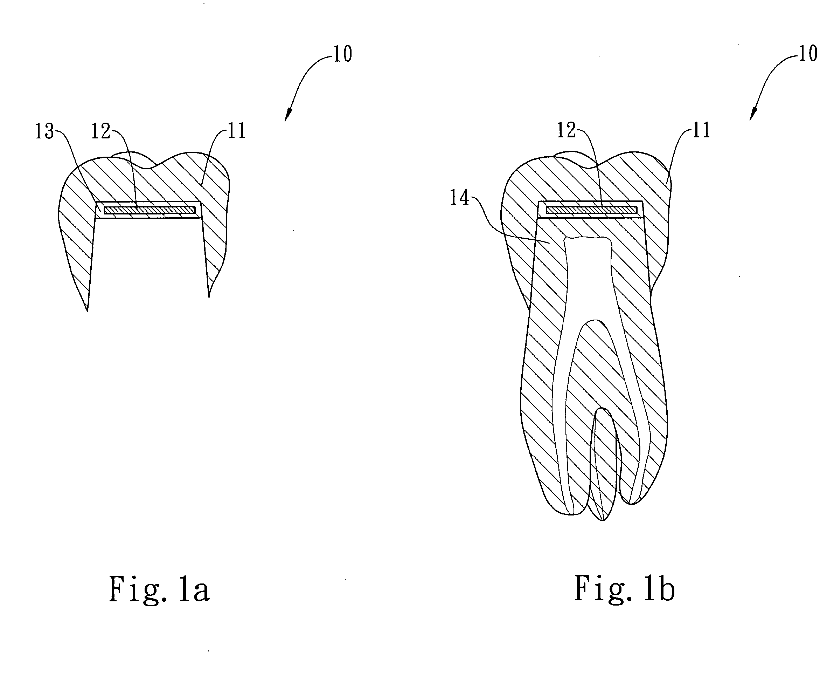

Please refer to figure 1a, said dental crown 11 was used as a carrier of the device 10 of the present invention. Next, a flash memory 12 stored with information was coated by a metal prosthesis 13 and then placed at the inner side of said dental crown 11. Please refer to figure 1b, said device 10 of the present invention was positioned on the ground original crown 14 to make said flash memory 12 hide inside the tooth of the user. The implantation of the device 10 of the present invention was accordingly completed.

Said information storage element of this example is a flash memory. Thus, when someone requires the information stored in said flash memory 12, it is needed to remove the device 10 of the present invention from the original crown 14 and take the flash memory 12 out from the inner side of said dental crown 11 to a reader (figure not shown) to read out the information therein for identify the user and other information thereof.

### Example 2: Implantation and read of the identification device of the present invention containing a dental post as an implantation carrier

Dental caries is a common dental disease. When a tooth is sulfuring a serious dental caries, a root canal treatment is often taken to treat the infected tooth for preventing the area infected by bacteria from broadening and worsening and reserving parts of the function of the tooth. A root canal treatment is what called "nerve- removing", but in fact, a complete root canal treatment comprises removing the entire slough including vessels, nerves and bacteria inside the tooth and leaving an empty pulp chamber (a root canal). As long as covering a dental crown, the tooth being root canal treated can still have some masticatory function. However, since the vessels and nerves have been removed, the treated tooth has lost its vitality and is much easier to be broken than a healthy tooth. In order to provide enough strength to support the treated tooth, a dental post is often positioned in said pulp chamber to improve the strength thereof, and the original crown is ground for placing a dental crown. Thus, the masticatory function of the treated tooth can be maintained for a longer time by assistance of the supportive strength rendered by said dental post and the protection rendered by said dental crown.

An information storage element used in this example was a contact chip with a short-tube shape and has a titanium-etched code. Said contact chip was stored with an ID number, name, birthday, blood type and record of dread diseases of the user.

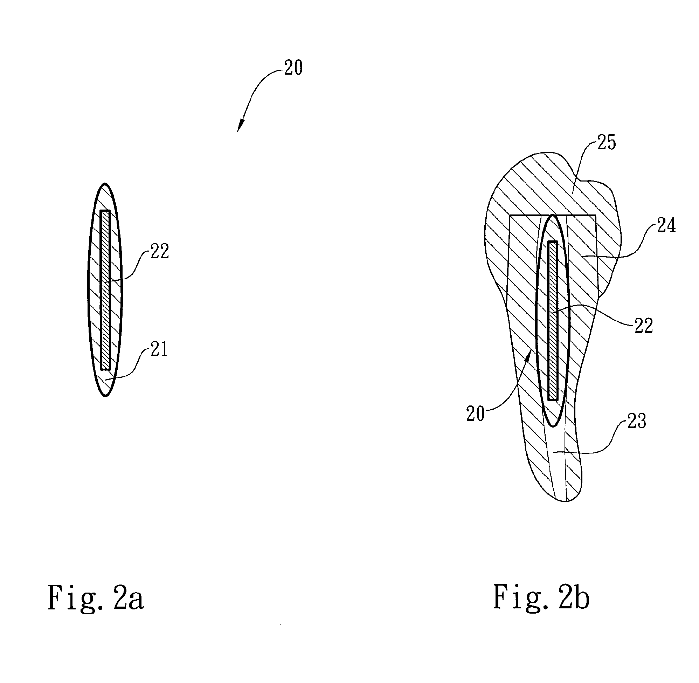

Please refer to figure 2a, a dental post 21 was used as a carrier of the device 20 of the present invention. A chip 22 stored with information was positioned inside said dental post 21, and said dental post 21 was made of pure titanium (titanium with a purity of at least 99%).

Please refer to figure 2b, in order to implant the device 20 of the present invention: first, a pulp chamber 23 of a tooth was cleaned completely, and the original crown 24 thereof was ground into a proper size for serving as a support for securing a dental crown 25. Then, a mold was sampled on said ground original crown 24 by well known operation, and a dental crown 25 was fabricated in accordance with said mold. Next, said device 20 of the present invention was positioned inside said pulp chamber 23 and said dental crown 25 was positioned on said original crown 24. Accordingly, the implantation of the device 20 of the present invention was completed.

The information storage element of this example comprises said contact chip 22 and said titanium-etched code (figure not shown). When someone requires the information stored in said chip 22, it is needed to remove said device 20 of the present invention from said pulp chamber 23 of the user's tooth and take out said chip 22 therein. Said chip 22 is then read in a reader (figure not shown) for identifying the user and information thereof. On the other hand, when said chip 22 is dysfunction, said titanium-etched code, which was etched on a surface of said chip 22, is read and entered in a predetermined database for identifying the user and obtaining information thereof.

### Example 3: Implantation and read of the identification device of the present invention containing a dental implant as an implantation carrier

When a life-span of a normal tooth is going to over, an artificial tooth is a necessity for satisfying the chewing demand of human beings. The technique of artificial teeth has been gradually improved and, improvements for lots of related components thereof are also existed. This example merely takes a conventional artificial tooth as an example to describe an embodiment of the present invention, which uses a dental implant as an implantation carrier.

An information storage element used in this example was a contactless chip in a short-tube shape and has a titanium-etched code. Said contactless chip was stored with an ID number of the user and a remaining sum of EasyCard.

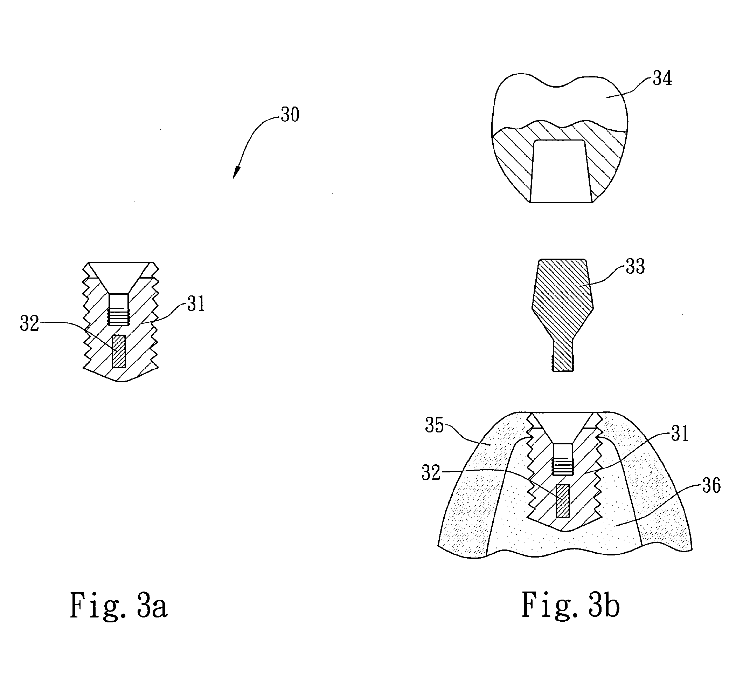

Please refer to figure 3a, a device 30 of the present invention comprised a dental implant 31 and a chip 32 stored with information. Said dental implant 31 was made of pure titanium (titanium with a purity of at least 99%), and said chip 32 stored with information was positioned inside said dental implant 31.

Please refer to figure 3b, in order to implant the device 30 of the present invention: first, an original tooth was extracted, and an aperture was drilled on an alveolar bone 36 thereof. Then, said dental implant 31 containing said chip 32 was positioned in said aperture. After waiting for a period of time for gums 35 to heal, the healed gums 35 was opened again to let said dental implant 31 therein expose for connecting a base 33. Next, a dental crown 34 was positioned on said base and the implantation of the device 30 was completed.

The information storage element of this example comprised said contactless chip 32 and said titanium-etched code (figure not shown), wherein said contactless chip 32 was stored with an ID number of the user and a remaining sum of EasyCard. When the user wants to use the EasyCard, he or she can just pass by a passage, which a sensor (figure not shown) for reading said chip 32 is equipped, and said sensor can read out the remaining sum recorded in said chip 32 and update a new remaining sum after carrying out a deduction. If adding value is needed, the user can simply stand beside an add-value machine equipped with a sensor for said sensor to read the information stored in said chip 32. Then the machine will show the ID number of the user in the display for the user to confirm if said chip 32 is read correctly. In the mean time, the machine can take actions of adding value per the use's operation and updating a new remaining sum.

When said contactless chip 32 is dysfunction, said device 30 of the present invention is removed from the alveolar bone 36 and said chip 32 therein is taken from said dental implant 31. Then the titanium-etched code (figure not shown) is read and entered to corresponding database to identify the user and a remaining sum of EasyCard. After identification, the ID number and the remaining sum can be stored in a new chip 32. Then the new chip 32 can be re-equipped in said alveolar bone 36 and used as usual.

### Example 4: Implantation and read of the identification device of the present invention containing a dental bone screw as an implantation carrier

A tiny bone screw (mini-screw) is a common dental medical tool, for instance, an orthodontic procedure may need a bone screw as an auxiliary tool, wherein said bone screw may be anchored at a jaw of a mandible and served as a point of application for metal wires to move teeth.

An information storage element used in this example was a contactless chip in a short-tube shape and has a titanium-etched code. Said contactless chip was stored with an ID number, name, birthday, blood type and record of dread diseases of the user.

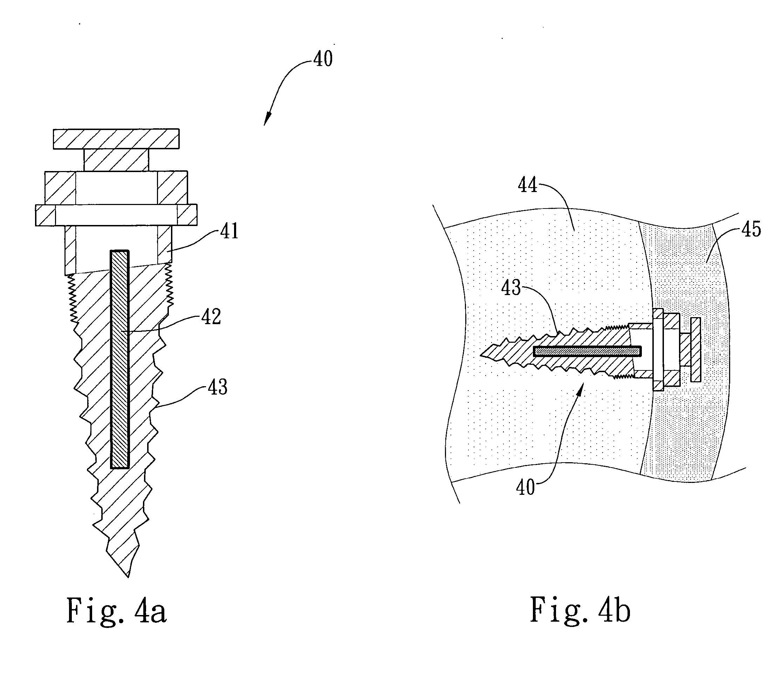

Please refer to figure 4a, a device 40 of the present invention comprised a bone screw 41 and a chip 42 stored with information positioned therein. Said bone screw 41 (mini-screw) was made of pure titanium and had a thread structure 43.

Please refer to figure 4b, said device 40 of the present invention was anchored at a predetermined site at a bone 44 in assistance of said thread structure 43 by a conventional dental operation. After a soft tissue (gums) 45 was healed, said device 40 of the present invention was completely embedded inside said soft tissue 45 and did not expose in the oral cavity.

There are at least two options for said predetermined site: 1) a site which is below the apex of mandibular incisor in a distance of 2mm and in front of the left and right mental foreman in a distance of at least 5mm; and 2) a site which is in an area from posterior buccal side of the mandibular 2nd molar to anterior buccal side of the ascending ramus.

Since the information storage element of this example comprised said contactless chip 42 and said titanium-etched code (figure not shown), when someone requires the information stored in said chip 42, a corresponding reader (figure not shown) is merely needed to read the information stored in said chip 42 from a proper distance for identifying the user and information thereof. When said chip 42 is dysfunction, said device 40 can be removed from said bone 44 of the user's oral cavity, and said chip 42 is taken from said bone screw 41 to recognize the titanium code etched on a surface of said chip 42. Then, said code is entered into a predetermined database to identify the user and information thereof.

The embodiments and the technical principles used are described above. All variations and modifications of the present invention and the uses thereof are included in the scope of the present invention if they do not depart from the spirit of the disclosure of this specification and drawings.

## Claims

1. An identification device, comprising:
a dental implantation carrier; and
an information storage element positioned in said implantation carrier.

2. The identification device according to claim 1, wherein said implantation carrier is a dental crown, a dental post, a dental implant or a bone screw.

3. The identification device according to claim 1, wherein said information comprises user identification, user personal information or a combination thereof.

4. The identification device according to claim 3, wherein said information further comprises a remaining sum of electronic money.

5. The identification device according to claim 1, wherein said information storage element is a chip, a flash memory, a metal-etched code or a combination thereof.

6. The identification device according to claim 5, wherein said chip is an identification chip.

7. The identification device according to claim 5, wherein said chip is a contactless chip, a contact chip or a combination thereof.

8. The identification device according to claim 1, wherein said information storage element is coated by a metal prosthesis.

9. The identification device according to claim 8, wherein a metal of said metal prosthesis is titanium.

10. A method for identification, comprising the following steps:
(a) positioning at least one identification device in accordance with claim 1 within an oral cavity of a subject to be identified; and
(b) reading an information storage element of said identification device.

11. The method according to claim 10, wherein said identification device is positioned within a tooth or a bone inside said oral cavity.

12. The method according to claim 10, further comprising a step of removing said identification device from said oral cavity before said step (b).

13. The method according to claim 10, wherein a type of said reading is contactless-reading or contact-reading.

14. The method according to claim 10, wherein said subject comprises human beings or animals.
